(19) Europäisches Patentamt
European Patent Office
Office européen des brevets

(11) Veröffentlichungsnummer: **0 397 005 A2**

(12) # EUROPÄISCHE PATENTANMELDUNG

(21) Anmeldenummer: **90108210.7**

(22) Anmeldetag: **30.04.90**

(51) Int. Cl.5: **C07D 249/08, C07D 233/60, C07D 233/61, A01N 43/653, A01N 43/50**

(30) Priorität: **06.05.89 DE 3914944**

(43) Veröffentlichungstag der Anmeldung: **14.11.90 Patentblatt 90/46**

(84) Benannte Vertragsstaaten: **AT BE CH DE DK ES FR GB GR IT LI NL SE**

(71) Anmelder: **BASF Aktiengesellschaft**
**Carl-Bosch-Strasse 38**
**D-6700 Ludwigshafen(DE)**

(72) Erfinder: **Seele, Rainer, Dr.**
**Leiblstrasse 3**
**D-6701 Fussgoenheim(DE)**
Erfinder: **Himmele, Walter, Dr.**
**Eichenweg 14**
**D-6909 Walldorf(DE)**
Erfinder: **Kober, Reiner, Dr.**
**Im Schlittweg 20**
**D-6701 Fussgoenheim(DE)**
Erfinder: **Ammermann, Eberhard, Dr.**
**Sachsenstrasse 3**
**D-6700 Ludwigshafen(DE)**
Erfinder: **Lorenz, Gisela, Dr.**
**Erlenweg 13**
**D-6730 Neustadt(DE)**
Erfinder: **Rademacher, Wilhelm, Dr.**
**Austrasse 1**
**D-6703 Limburgerhof(DE)**
Erfinder: **Jung, Johann, Dr.**
**Hardenburgstrasse 19**
**D-6703 Limburgerhof(DE)**

(54) **Azolylethanderivate und diese enthaltende Fungizide und Wachstumsregulatoren.**

(57) Azolylethanderivate der allgemeinen Formel I

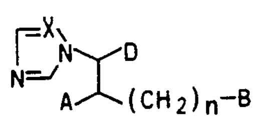

I

in welcher
A und B Alkyl, Phenyl, Biphenyl, Naphthyl, Benzyl, Cycloalkyl oder Cycloalkenyl bedeuten, wobei diese Reste substituiert sein können,
D Alkoxy, Amino, Mercapto, Thiophenyl, Aminophenyl, Hetaryloxy oder Phenoxy bedeutet, wobei diese Reste substituiert sein können,
n eine ganze Zahl von 1 bis 5 oder 0 bedeutet,
X den Rest CH oder N bedeutet,
sowie deren für Pflanzen verträgliche Säureadditionssalze und Metallkomplexe und diese Verbindungen enthaltende Fungizide und Wachstumsregulatoren.

EP 0 397 005 A2

## Azolylethanderivate und diese enthaltende Fungizide und Wachtumsregulatoren

Die vorliegende Erfindung betrifft neue Azolverbindungen, Verfahren zu ihrer Herstellung und diese enthaltende Fungizide und Wachstumsregulatoren.

Es ist bekannt, Triazolylethanderivate, z.B. das 1-(1,2,4-Triazol-1-yl)--1-phenylthio-2-methyl-2-phenylpropan oder das 1-(1,2,4-Triazol-1-yl)-1-(4-chlorphenylthio)-2-methyl-2-phenylpropan als Fungizide zu verwenden (EP-91219). Ihre fungizide Wirkung ist unbefriedigend.

Es ist ferner bekannt, Triazolylalkoxybenzylderivate als Fungizide zu verwenden (DE-26 40 823).

Es wurde nun gefunden, daß Azolylethanderivate der allgemeinen Formel I

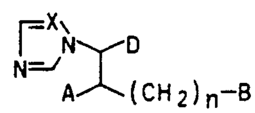

I

in welcher

A und B $C_1$-$C_8$-Alkyl, Phenyl, Biphenyl, Naphthyl, Benzyl, Tetrahydropyranyl, $C_3$-$C_8$-Cycloalkyl oder $C_5$-$C_8$-Cycloalkenyl bedeuten,

wobei diese Reste einfach bis dreifach durch Halogen, Nitro, Phenoxy, Amino, Alkyl, Alkoxy oder Halogenalkyl mit jeweils 1-bis 4-C-Atomen substituiert sein können,

$C_1$-$C_4$-Alkoxy, Amino, gegebenenfalls durch $C_1$-$C_3$-Alkyl, $C_1$-$C_3$-Acyl substituiertes Amino, gegebenenfalls durch $C_1$-$C_4$-Alkyl substituiertes Mercapto, Thiophenyl, Aminophenyl, Hetaryloxy oder Phenoxy bedeutet, wobei diese Reste einfach bis dreifach durch Halogen, Nitro, Phenoxy, Amino, Alkyl oder Halogenalkyl mit jeweils 1 bis 4 C-Atomen substituiert sein können,

n eine ganze Zahl von 1 bis 5 oder 0 bedeutet,

X den Rest CH oder N bedeutet,

sowie deren für Pflanzen verträgliche Säureadditionssalze oder Metallkomplexe, außer den Verbindungen, in denen A unsubstituiertes Phenyl, B Alkyl, D Alkoxy, X N und n 0 bedeuten, eine bessere fungizide Wirkung besitzen als bekannte Azolverbindungen.

Die Verbindungen der Formel I enthalten asymmetrische C-Atome und können daher als Enantiomere und Diastereomere auftreten. Die Gemische von Diastereomeren lassen sich bei den erfindungsgemäßen Verbindungen in üblicher Weise, beispielsweise aufgrund ihrer unterschiedlichen Löslichkeit oder durch Säulenchromatographie trennen und in reiner Form isolieren. Die Racemate lassen sich bei den erfindungsgemäßen Verbindungen nach bekannten Methoden, beispielsweise durch Salzbildung mit einer optisch aktiven Säure, Trennung der diastereomeren Salze und Freisetzung der Enantiomeren mittels einer Base trennen. Als fungizide und wachstumsregulatorische Wirkstoffe können sowohl die einzelnen Enantiomeren oder Diastereomeren als auch deren Gemische verwendet werden.

A und B sind gleich oder verschieden und bedeuten beispielsweise $C_1$-$C_4$-Alkyl, Methyl, Ethyl, Isopropyl, n-Propyl, n-Butyl, sec-Butyl, tert.-Butyl, n-Pentyl, Neopentyl, 1-Naphthyl, 2-Naphthyl, p-Biphenyl, Phenyl, 2-Chlorphenyl, 2-Fluorphenyl, 2-Bromphenyl, 3-Chlorphenyl, 3-Bromphenyl, 3-Fluorphenyl, 4-Fluorphenyl, 4-Chlorphenyl, 4-Bromphenyl, 2,4-Dichlorphenyl, 2,3-Dichlorphenyl, 2,5-Dichlorphenyl, 2,6-Dichlorphenyl, 2-Chlor-6-fluorphenyl, 2-Methoxyphenyl, 3-Methoxyphenyl, 4-Methoxyphenyl, 2,4-Dimethoxyphenyl, 4-Ethylphenyl, 4-Isopropylphenyl, 4-tert.-Butylphenyl, 4-tert.Butyloxyphenyl, 2-Chlor-4-fluorphenyl, 2-Chlor-6-methylphenyl, 3,4-Dimethoxyphenyl, 3-Phenoxyphenyl, 4-Phenoxyphenyl, 3-Nitrophenyl, 4-Nitrophenyl, 3-Aminophenyl, 4-Aminophenyl, 2-Aminophenyl, 2-Trifluormethylphenyl, 3-Trifluormethylphenyl, 4-Trifluormethylphenyl, Tetrahydropyranyl, Cyclopropyl, Cyclopentyl, Cyclohexyl, 2-Cyclohexenyl, 3-Cyclohexenyl.

n bedeutet 0, 1, 2, 3, 4 oder 5

Säureadditionssalze sind beispielsweise die Hydrochloride, Bromide, Sulfate, Nitrate, Phosphate, Oxalate oder Dodecylbenzolsulfonate. Die Wirksamkeit der Salze geht auf das Kation zurück, so daß es auf das Anion i.a. nicht ankommt. Die erfindungsgemäßen Wirkstoffsalze werden hergestellt durch Umsetzung der Azolylethanderivate (I) mit den Säuren.

Metallkomplexe der Wirkstoffe I oder ihrer Salze können mit Kupfer, Zink, Zinn, Mangan, Eisen, Kobalt oder Nickel gebildet werden, indem man die Azolylethanderivate mit entsprechenden Metallsalzen umsetzt.

Die Verbindungen der Formel I können hergestellt werden, indem man eine Verbindung der Formel II

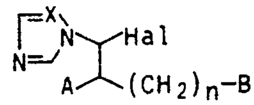

II

in welcher
A, B und X die oben angegebene Bedeutung haben und Hal den Rest Chlor oder Brom bedeutet, mit einer Verbindung der Formel D-H umsetzt.

Die Reaktion erfolgt gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 10 und 150°C. Zu den bevorzugten Lösungs-und Verdünnungsmitteln gehören Ketone wie Aceton, Methylethylketon oder Cyclohexanon, Nitrile wie Acetonitril oder Propionitril, Alkohole wie Methanol, Ethanol, iso-Propanol, n-Butanol oder Glycol, Ester wie Essigsäureethylester, Essigsäuremethylester oder Essigsäurebutylester, Ether wie Tetrahydrofuran, Diethylether, Dimethoxyethan, Dioxan oder Diisopropylether, Amide wie Dimethylformamid oder N-Methylpyrrolidon, ferner Dimethylsulfoxid, Sulfolan oder entsprechende Gemische.

Geeignete Basen, die gegebenenfalls auch als säurebindende Mittel bei der Reaktion verwendet werden können, sind beispielsweise Alkalihydroxide wie Lithium-, Natrium- oder Kaliumhydroxid, Alkalicarbonate wie Natrium-, Kalium- oder Caesiumcarbonat, Alkalihydride wie Lithium-, Natrium- und Kaliumhydrid, Alkaliamide wie Natrium- und Kaliumamid, ferner Lithium-, Natrium-oder Kalium-triphenylmethyl und Naphthalinlithium, -natrium oder -kalium, Pyridin oder 4-Dimethylaminopyridin. Es können aber auch andere übliche Basen verwendet werden.

Als Reaktionsbeschleuniger kommen vorzugsweise Metallhalogenide wie Natriumjodid oder Kaliumjodid, quarternäre Ammoniumsalze wie Tetrabutylammoniumchlorid, -bromid, -jodid oder -hydrogensulfat, Benzyltriethylammoniumchlorid oder -bromid oder Kronenether wie 12-Krone-4, 15-Krone-5, 18-Krone-6, Dibenzo-18-Krone-6 oder dicyclohexano-18-Krone-6 in Frage.

Die Umsetzung wird im allgemeinen bei Temperaturen zwischen 20 und 150°C, drucklos oder unter Druck, kontinuierlich oder diskontinuierlich durchgeführt.

Die Ausgangsverbindungen II können hergestellt werden, indem man eine Verbindung der Formel III

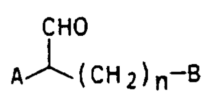

III

in welcher
A, B und n die oben angegebene Bedeutung haben, mit einer Verbindung der Formel IV

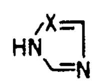

IV

in der
X die angegebene Bedeutung hat, in Gegenwart von Thionylhalogeniden zur Umsetzung bringt.

Die Umsetzung erfolgt gegebenenfalls in Gegenwart eines Lösungs- oder Verdünnungsmittels bei Temperaturen zwischen -30 und 80°C. Zu den bevorzugten Lösungs- oder Verdünnungsmitteln gehören Nitrile wie Acetonitril oder Propionitril, Ether wie Tetrahydrofuran, Diethylether, Dimethoxyethan, Dioxan oder Diisopropylether und insbesondere Kohlenwasserstoffe und Chlorkohlenwasserstoffe wie Pentan, Hexan, Methylenchlorid, Chloroform, Tetrachlorkohlenstoff, Dichlorethan oder entsprechende Gemische.

Die Verbindungen III lassen sich entsprechend allgemein bekannter Verfahren zur Aldehydsynthese (Houben-Weyl-Müller, Methoden der Organischen Chemie, Georg Thieme Verlag, Stuttgart 1983, Bd. E) herstellen.

Die folgenden Beispiele erläutern die Herstellung der Wirkstoffe.

1. Herstellung der Ausgangsstoffe

Vorschrift 1

1-(1,2,4-Triazol-1-yl)-1-chlor-2-(2,4-dichlorphenyl)-butan

Zu einer Lösung von 307,9 g Triazol in 500 ml Methylenchlorid werden bei 0°C 132,8 g Thionylchlorid zugesetzt. Nach beendeter Zugabe wird die Mischung bei Raumtemperatur (20°C) für 30 Minuten gerührt und anschließend 116,3 g 2-(2,4-dichlorphenyl)-butanal zugegeben. Nachdem das Reaktionsgemisch 15 Stunden bei Raumtemperatur rührte, werden der Lösung 400 ml Wasser zugesetzt und die organische Phase abgetrennt. Die verbleibende wäßrige Phase wird zweimal mit Methylenchlorid ausgeschüttelt und die gesammelten organischen Phasen zweimal mit gesättigter Natriumhydrogencarbonat-Lösung gewaschen. Die isolierte organische Phase wird daraufhin über Natriumsulfat getrocknet und eingeengt, wobei 158,2 g 1-(1,2,4-Triazol-1-yl)-1-chlor-2-(2,4-dichlorphenyl)-butan erhalten werden.

2. Herstellung der Endprodukte

Beispiel 1

1-(1,2,4-Triazol-1-yl)-1-methoxy2-(2,4-dichlorphenyl)-butan

Zu einer Lösung von 26,6 g 1-(1,2,4-Triazol-1-yl)-1-chlor-2-(2,4-dichlorphenyl)-butan in 200 ml Methanol werden 9,4 g Natriummethylat und 0,2 g Kaliumjodid gegeben. Nachdem das Reaktionsgemisch 48 Stunden unter Rückfluß erhitzt wurde, wird der Lösung 100 ml Wasser zugesetzt und mehrmals mit Methyl-tert.-butylether extrahiert. Die isolierte organische Phase wird zweimal mit Wasser gewaschen, daraufhin über Natriumsulfat getrocknet, eingeengt und der verbleibende Rückstand durch Chromatographie an Kieselgel (Essigester n-Hexan = 9:1) gereinigt. Man erhält 6,2 g (24 %) 1-(1,2,4-Triazol-1-yl)-1-methoxy-2-(2,4-dichlorphenyl)-butan als 1:1-Diastereomerengemisch (Verbindung Nr. 1).

Entsprechend Beispiel 1 können die in der Tabelle aufgeführten Verbindungen hergestellt werden.

Tabelle zum Neuanmeldungsentwurf Fungizide Azolylethanderivate

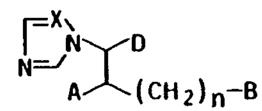

I

| Bsp | A | B | D | n | X | Schmp./IR ($cm^{-1}$) | Isomer* |
|---|---|---|---|---|---|---|---|
| 1 | $2,4-Cl_2-C_6H_3$ | $CH_3$ | $OCH_3$ | 1 | N | 2968, 1502, 1475, 1275 1135, 1103, 806 $cm^{-1}$ | $D_1:D_2=1:1$ |
| 2 | $2,4-Cl_2-C_6H_3$ | $CH_3$ | $OCH_3$ | 1 | CH | | |
| 3 | $2,4-Cl_2-C_6H_3$ | $CH_3$ | $OC_2H_5$ | 1 | N | | |
| 4 | $2,4-Cl_2-C_6H_3$ | $CH_3$ | 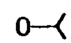 | 1 | N | | |
| 5 | $2,4-Cl_2-C_6H_3$ | $CH_3$ | $S-CH_3$ | 1 | N | | |
| 6 | $2,4-Cl_2-C_6H_3$ | $CH_3$ | $S-CH_3$ | 1 | CH | | |
| 7 | $2,4-Cl_2-C_6H_3$ | $CH_3$ | $NHCH_3$ | 1 | N | | |
| 8 | $2,4-Cl_2-C_6H_3$ | $CH_3$ | $N(CH_3)_2$ | 1 | N | | |
| 9 | $2,4-Cl_2-C_6H_3$ | $CH_3$ | $NH_2$ | 1 | N | | |
| 10 | $2,4-Cl_2-C_6H_3$ | $CH_3$ | $S-C_6H_5$ | 1 | N | | |
| 11 | $2,4-Cl_2-C_6H_3$ | $CH_3$ | $NH-C_6H_5$ | 1 | N | | |
| 12 | $2,4-Cl_2-C_6H_3$ | $CH_3$ | $O-C_6H_5$ | 1 | N | | |
| 13 | $2,4-Cl_2-C_6H_3$ | $CH_3$ | 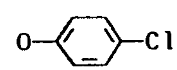 | 1 | N | | |

Tabelle (Fortsetzung)

| Bsp | A | B | D | n | X | Schmp./IR ($cm^{-1}$) | Isomer* |
|---|---|---|---|---|---|---|---|
| 14 | 2,4-$Cl_2$-$C_6H_3$ | $CH_3$ | 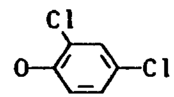 | 1 | N | | |
| 15 | 2,4-$Cl_2$-$C_6H_3$ | $CH_3$ | 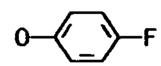 | 1 | N | | |
| 16 | 2,4-$Cl_2$-$C_6H_3$ | $CH_3$ | $OCH_3$ | 0 | N | | |
| 17 | 2,4-$Cl_2$-$C_6H_3$ | $CH_3$ | $OCH_3$ | 0 | CH | | |
| 18 | 2,4-$Cl_2$-$C_6H_3$ | $CH_3$ | $OC_2H_5$ | 0 | N | | |
| 19 | 2,4-$Cl_2$-$C_6H_3$ | $CH_3$ | $OC_2H_5$ | 0 | CH | | |
| 20 | 2,4-$Cl_2$-$C_6H_3$ | $CH_3$ | S-$CH_3$ | 0 | N | | |
| 21 | 2,4-$Cl_2$-$C_6H_3$ | $CH_3$ | NH-$COCH_3$ | 1 | N | | |
| 22 | 2,4-$Cl_2$-$C_6H_3$ | $CH_3$ | $NH_2$ | 0 | N | | |
| 23 | 2,4-$Cl_2$-$C_6H_3$ | $CH_3$ | 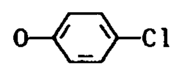 | 0 | N | | |
| 24 | 2,4-$Cl_2$-$C_6H_3$ | $CH_3$ | O-$C_6H_5$ | 0 | N | | |
| 25 | 2,4-$Cl_2$-$C_6H_3$ | $CH_3$ | 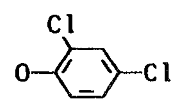 | 0 | N | | |
| 26 | 2,4-$Cl_2$-$C_6H_3$ | $CH_3$ | 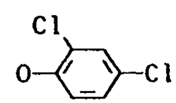 | 0 | CH | | |

Tabelle (Fortsetzung)

| Bsp | A | B | D | n | X | Schmp./IR ($cm^{-1}$) | Isomer* |
|---|---|---|---|---|---|---|---|
| 27 | $2,4-Cl_2-C_6H_3$ | $CH_3$ | $OCH_3$ | 2 | N | 1960,1502,1475,1275 1134,1103,823 $cm^{-1}$ | $D_1:D_2=1:1$ |
| 28 | $2,4-Cl_2-C_6H_3$ | $CH_3$ | $OCH_3$ | 2 | CH | | |
| 29 | $2,4-Cl_2-C_6H_3$ | $CH_3$ | $OC_2H_5$ | 2 | N | | |
| 30 | $2,4-Cl_2-C_6H_3$ | $CH_3$ | $OC_2H_5$ | 2 | CH | | |
| 31 | $2,4-Cl_2-C_6H_3$ | $CH_3$ | 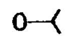 | 2 | N | | |
| 32 | $2,4-Cl_2-C_6H_3$ | $CH_3$ | $SCH_3$ | 2 | N | | |
| 33 | $2,4-Cl_2-C_6H_3$ | $CH_3$ | $S-C_3H_7$ | 2 | N | | |
| 34 | $2,4-Cl_2-C_6H_3$ | $CH_3$ | $NH_2$ | 2 | N | | |
| 35 | $2,4-Cl_2-C_6H_3$ | $CH_3$ | $S-C_6H_5$ | 2 | N | 75-78°C | Enantiomerengemisch |
| 36 | $2,4-Cl_2-C_6H_3$ | $CH_3$ | $NH-C_6H_5$ | 2 | N | | |
| 37 | $2,4-Cl_2-C_6H_3$ | $CH_3$ | $O-C_6H_5$ | 2 | N | | |
| 38 | $2,4-Cl_2-C_6H_3$ | $CH_3$ | 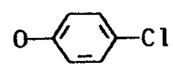 | 2 | N | | |
| 39 | $2,4-Cl_2-C_6H_3$ | $CH_3$ | 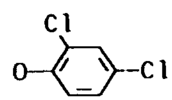 | 2 | N | | |
| 40 | $2,4-Cl_2-C_6H_3$ | $CH_3$ | 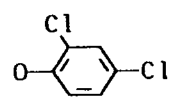 | 2 | CH | | |
| 41 | $2,4-Cl_2-C_6H_3$ | $CH_3$ | 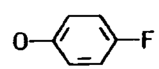 | 2 | N | | |

Tabelle (Fortsetzung)

| Bsp | A | B | D | n | X | Schmp./IR ($cm^{-1}$) | Isomer* |
|---|---|---|---|---|---|---|---|
| 42 | 2,4-$Cl_2$-$C_6H_3$ | $CH_3$ | $OCH_3$ | 3 | N | 2957, 1501, 1475, 1275 1104, 809 $cm^{-1}$ | $D_1$:$D_2$=1,2:1 |
| 43 | 2,4-$Cl_2$-$C_6H_3$ | $CH_3$ | $OCH_3$ | 3 | CH | | |
| 44 | 2,4-$Cl_2$-$C_6H_3$ | $CH_3$ | $OC_2H_5$ | 3 | N | 2957, 1500, 1474, 1274 1132, 1103, 807 $cm^{-1}$ | $D_1$:$D_2$=1:1 |
| 45 | 2,4-$Cl_2$-$C_6H_3$ | $CH_3$ | $OC_4H_9$ | 3 | N | | |
| 46 | 2,4-$Cl_2$-$C_6H_3$ | $CH_3$ | $SCH_3$ | 3 | N | | |
| 47 | 2,4-$Cl_2$-$C_6H_3$ | $CH_3$ | $N(CH_3)_2$ | 3 | N | | |
| 48 | 2,4-$Cl_2$-$C_6H_3$ | $CH_3$ | O-$C_6H_5$ | 3 | N | | |
| 49 | 2,4-$Cl_2$-$C_6H_3$ | $CH_3$ | 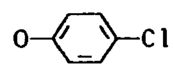 | 3 | N | 2957, 1504, 1490, 1475, 1275, 1135, 825, 805 $cm^{-1}$ | $D_1$:$D_2$=1:1 |
| 50 | 2,4-$Cl_2$-$C_6H_3$ | $CH_3$ | 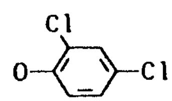 | 3 | N | | |
| 51 | 2,4-$Cl_2$-$C_6H_3$ | $CH_3$ | S-$C_6H_5$ | 3 | N | | |
| 52 | 2,4-$Cl_2$-$C_6H_3$ | $CH_3$ | $OCH_3$ | 4 | N | | |
| 53 | 2,4-$Cl_2$-$C_6H_3$ | $CH_3$ | $OCH_3$ | 4 | CH | | |
| 54 | 2,4-$Cl_2$-$C_6H_3$ | $CH_3$ | $OCH_3$ | 5 | N | | |
| 55 | 2,4-$Cl_2$-$C_6H_3$ | $CH_3$ | $OCH_3$ | 5 | CH | | |
| 56 | $C_6H_5$ | $CH_3$ | $OCH_3$ | 0 | N | | |
| 57 | $C_6H_5$ | $CH_3$ | $OCH_3$ | 0 | CH | | |

Tabelle (Fortsetzung)

| Bsp | A | B | D | n | X | Schmp./IR ($cm^{-1}$) | Isomer* |
|---|---|---|---|---|---|---|---|
| 58 | $C_6H_5$ | $CH_3$ | $NH_2$ | 0 | N | | |
| 59 | $C_6H_5$ | $CH_3$ | $SCH_3$ | 0 | N | | |
| 60 | $C_6H_5$ | $CH_3$ | $OC_6H_5$ | 0 | N | | |
| 61 | $C_6H_5$ | $CH_3$ | $SC_6H_5$ | 0 | N | | |
| 62 | $C_6H_5$ | $CH_3$ | 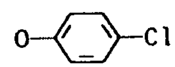 | 0 | N | | |
| 63 | $C_6H_5$ | $CH_3$ | 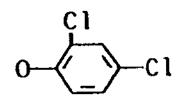 | 0 | N | | |
| 64 | $C_6H_5$ | $CH_3$ | $OCH_3$ | 1 | N | 1501, 1274, 1135, 701; | $D_1:D_2$ = 1:1 |
| 65 | $C_6H_5$ | $CH_3$ | $OC_2H_5$ | 1 | N | | |
| 66 | $C_6H_5$ | $CH_3$ | $SCH_3$ | 1 | N | | |
| 67 | $C_6H_5$ | $CH_3$ | 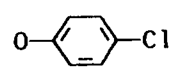 | 1 | N | | |
| 68 | $C_6H_5$ | $CH_3$ | $OCH_3$ | 2 | N | | |
| 69 | $C_6H_5$ | $CH_3$ | $OC_2H_5$ | 2 | N | | |
| 70 | $C_6H_5$ | $CH_3$ | 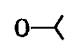 | 2 | N | | |
| 71 | $C_6H_5$ | $CH_3$ | $SCH_3$ | 2 | N | | |

Tabelle (Fortsetzung)

| Bsp | A | B | D | n | X | Schmp./IR ($cm^{-1}$) | Isomer* |
|---|---|---|---|---|---|---|---|
| 72 | $C_6H_5$ | $CH_3$ | $NH-C_6H_5$ | 2 | N | | |
| 73 | $C_6H_5$ | $CH_3$ | 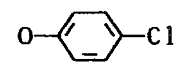 | 2 | N | | |
| 74 | $C_6H_5$ | $CH_3$ | $OCH_3$ | 3 | N | 2955, 2932, 1498, 1274, 1135, 1102, 701 $cm^{-1}$ | $D_1:D_2$ = 1:1 |
| 75 | $C_6H_5$ | $CH_3$ | $OC_2H_5$ | 3 | N | | |
| 76 | $C_6H_5$ | $CH_3$ | $SCH_3$ | 3 | N | | |
| 77 | $C_6H_5$ | $CH_3$ | 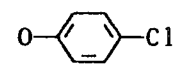 | 3 | N | | |
| 78 | $C_6H_5$ | $CH_3$ | $OCH_3$ | 4 | N | | |
| 79 | $C_6H_5$ | $CH_3$ | $OCH_3$ | 5 | N | 2928, 1499, 1274, 1135, 701 | $D_1:D_2$ = 1:1 |
| 80 | $C_6H_5$ | $C_6H_5$ | $OCH_3$ | 1 | N | | |
| 81 | $C_6H_5$ | $C_6H_5$ | $OC_2H_5$ | 1 | N | | |
| 82 | $C_6H_5$ | $C_6H_5$ | $SCH_3$ | 1 | N | | |
| 83 | $C_6H_5$ | $C_6H_5$ | 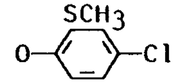 | 1 | N | | |
| 84 | $C_6H_5$ | $C_{10}H_7$ | $OCH_3$ | 1 | N | | |
| 85 | $C_6H_5$ | $C_{12}H_9$ | $OCH_3$ | 1 | N | | |
| 86 | $C_6H_5$ | 2,4-$Cl_2$-$C_6H_3$ | $OCH_3$ | 1 | N | | |
| 87 | $C_6H_5$ | 2,4-$Cl_2$-$C_6H_3$ | $OCH_3$ | 2 | N | | |

Tabelle (Fortsetzung)

| Bsp | A | B | D | n | X | Schmp./IR ($cm^{-1}$) | Isomer* |
|---|---|---|---|---|---|---|---|
| 88 | $C_6H_5$ | 4-F-$C_6H_4$ | $OCH_3$ | 1 | N | | |
| 89 | $C_6H_5$ | 2-Cl-$C_6H_4$ | $OCH_3$ | 1 | N | | |
| 90 | $C_6H_5$ | 4-Cl-$C_6H_4$ | $OCH_3$ | 1 | N | | |
| 91 | $C_6H_5$ | Cyclohexyl | $OCH_3$ | 1 | N | | |
| 92 | $C_6H_5$ | Cyclohexyl | $OCH_3$ | 1 | CH | | |
| 93 | $C_6H_5$ | Cyclohexyl | $OCH_3$ | 0 | N | | |
| 94 | $C_6H_5$ | Cyclohexyl | $SCH_3$ | 0 | N | | |
| 95 | $C_6H_5$ | Cyclohexyl | 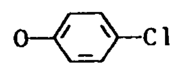 | 0 | N | | |
| 96 | 4-F-$C_6H_4$ | iso-$C_3H_7$ | $OCH_3$ | 0 | N | | |
| 97 | 4-F-$C_6H_4$ | iso-$C_3H_7$ | $OC_2H_5$ | 0 | N | | |
| 98 | 4-F-$C_6H_4$ | iso-$C_3H_7$ | $SCH_3$ | 0 | N | | |
| 99 | 4-F-$C_6H_4$ | iso-$C_3H_7$ | $OC_6H_5$ | 0 | N | | |
| 100 | 4-F-$C_6H_4$ | iso-$C_3H_7$ | 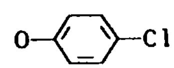 | 0 | N | | |
| 101 | 4-F-$C_6H_4$ | Cyclohexyl | $OCH_3$ | 0 | N | | |
| 102 | 4-F-$C_6H_4$ | 3-Cyclohexyl | $OCH_3$ | 0 | N | | |
| 103 | 4-F-$C_6H_4$ | $CH_3$ | $OCH_3$ | 0 | N | | |
| 104 | 4-F-$C_6H_4$ | $CH_3$ | $OC_2H_5$ | 0 | N | | |
| 105 | 4-F-$C_6H_4$ | $CH_3$ | $SCH_3$ | 0 | N | | |
| 106 | 4-F-$C_6H_4$ | $CH_3$ | 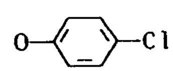 | 0 | N | | |

Tabelle (Fortsetzung)

| Bsp | A | B | D | n | X | Schmp./IR ($cm^{-1}$) | Isomer* |
|---|---|---|---|---|---|---|---|
| 107 | 4-F-$C_6H_4$ | $CH_3$ | $OCH_3$ | 1 | N | | |
| 108 | 4-F-$C_6H_4$ | $CH_3$ | $SCH_3$ | 1 | N | | |
| 109 | 4-F-$C_6H_4$ | $CH_3$ | $OC_6H_5$ | 1 | N | | |
| 110 | 4-F-$C_6H_4$ | $CH_3$ | $SC_6H_5$ | 1 | N | | |
| 111 | 4-F-$C_6H_4$ | $CH_3$ | 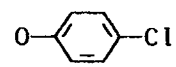 | 1 | N | | |
| 112 | 4-F-$C_6H_4$ | $CH_3$ | $OCH_3$ | 2 | N | | |
| 113 | 4-F-$C_6H_4$ | $CH_3$ | $SCH_3$ | 2 | N | | |
| 114 | 4-F-$C_6H_4$ | $CH_3$ | $HNC_6H_5$ | 2 | N | | |
| 115 | 4-F-$C_6H_4$ | $CH_3$ | $OCH_3$ | 3 | N | | |
| 116 | 4-F-$C_6H_4$ | $CH_3$ | $SCH_3$ | 3 | N | | |
| 117 | 4-F-$C_6H_4$ | $CH_3$ | $OCH_3$ | 5 | N | | |
| 118 | 4-F-$C_6H_4$ | $C_6H_5$ | $OCH_3$ | 1 | N | | |
| 119 | 4-F-$C_6H_4$ | 4-Cl-$C_6H_4$ | $OCH_3$ | 1 | N | | |
| 120 | 4-F-$C_6H_4$ | 4-F-$C_6H_4$ | $OCH_3$ | 1 | N | | |
| 121 | 4-F-$C_6H_4$ | 4-$OCH_3$-$C_6H_4$ | $OCH_3$ | 1 | N | | |
| 122 | 4-F-$C_6H_4$ | 2-Cl-$C_6H_4$ | $OCH_3$ | 1 | N | | |
| 123 | 4-Cl-$C_6H_4$ | iso-$C_3H_7$ | $OCH_3$ | 1 | N | | |
| 124 | 4-Cl-$C_6H_4$ | iso-$C_3H_7$ | $SCH_3$ | 1 | N | | |
| 125 | 4-Cl-$C_6H_4$ | iso-$C_3H_7$ | 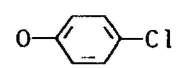 | 1 | N | | |
| 126 | 4-Cl-$C_6H_4$ | Cyclohexyl | $OCH_3$ | 0 | N | | |

Tabelle (Fortsetzung)

| Bsp | A | B | D | n | X | Schmp./IR ($cm^{-1}$) | Isomer* |
|---|---|---|---|---|---|---|---|
| 127 | 4-Cl-$C_6H_4$ | 2-Cyclohexenyl | $OCH_3$ | 0 | N | | |
| 128 | 4-Cl-$C_6H_4$ | $CH_3$ | $OCH_3$ | 0 | N | | |
| 129 | 4-Cl-$C_6H_4$ | $CH_3$ | $OC_2H_5$ | 0 | N | | |
| 130 | 4-Cl-$C_6H_4$ | $CH_3$ | $SCH_3$ | 0 | N | | |
| 131 | 4-Cl-$C_6H_4$ | $CH_3$ | 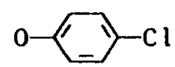 | 0 | N | | |
| 132 | 4-Cl-$C_6H_4$ | $CH_3$ | $OCH_3$ | 1 | N | | |
| 133 | 4-Cl-$C_6H_4$ | $CH_3$ | $OC_6H_5$ | 1 | N | | |
| 134 | 4-Cl-$C_6H_4$ | $CH_3$ | $SC_6H_5$ | 1 | N | | |
| 135 | 4-Cl-$C_6H_4$ | $CH_3$ | 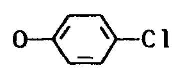 | 1 | N | | |
| 136 | 4-Cl-$C_6H_4$ | $CH_3$ | $OCH_3$ | 2 | N | | |
| 137 | 4-Cl-$C_6H_4$ | $CH_3$ | $SCH_3$ | 2 | N | | |
| 138 | 4-Cl-$C_6H_4$ | $CH_3$ | $N(CH_3)_2$ | 2 | N | | |
| 139 | 4-Cl-$C_6H_4$ | $CH_3$ | $OCH_3$ | 3 | N | | |
| 140 | 4-Cl-$C_6H_4$ | $CH_3$ | $SCH_3$ | 3 | N | | |
| 141 | 4-Cl-$C_6H_4$ | $CH_3$ | $OCH_3$ | 5 | N | | |
| 142 | 4-Cl-$C_6H_4$ | $C_6H_5$ | $OCH_3$ | 1 | N | | |
| 143 | 4-Cl-$C_6H_4$ | 4-F-$C_6H_4$ | $OCH_3$ | 1 | N | | |
| 144 | 4-Cl-$C_6H_4$ | 2-Cl-$C_6H_4$ | $OCH_3$ | 1 | N | | |
| 145 | 4-Br-$C_6H_4$ | iso-$C_3H_7$ | $OCH_3$ | 1 | N | | |
| 146 | 4-Br-$C_6H_4$ | Cyclohexyl | $OCH_3$ | 1 | N | | |

Tabelle (Fortsetzung)

| Bsp | A | B | D | n | X | Schmp./IR ($cm^{-1}$) | Isomer* |
|---|---|---|---|---|---|---|---|
| 147 | 4-Br-$C_6H_4$ | $CH_3$ | $OCH_3$ | 0 | N | | |
| 148 | 4-Br-$C_6H_4$ | $CH_3$ | $OC_2H_5$ | 0 | N | | |
| 149 | 4-Br-$C_6H_4$ | $CH_3$ | $SCH_3$ | 0 | N | | |
| 150 | 4-Br-$C_6H_4$ | $CH_3$ | 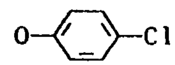 | 0 | N | | |
| 151 | 4-Br-$C_6H_4$ | $CH_3$ | $OCH_3$ | 1 | N | | |
| 152 | 4-Br-$C_6H_4$ | $CH_3$ | $OC_2H_5$ | 1 | N | | |
| 153 | 4-Br-$C_6H_4$ | $CH_3$ | $SC_6H_5$ | 1 | N | | |
| 154 | 4-Br-$C_6H_4$ | $CH_3$ | 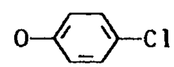 | 1 | N | | |
| 155 | 4-Br-$C_6H_4$ | $CH_3$ | $OCH_3$ | 2 | N | | |
| 156 | 4-Br-$C_6H_4$ | $CH_3$ | $SCH_3$ | 2 | N | | |
| 157 | 4-Br-$C_6H_4$ | $CH_3$ | NH-$C_6H_5$ | 2 | N | | |
| 158 | 4-Br-$C_6H_4$ | $CH_3$ | $OCH_3$ | 3 | N | | |
| 159 | 4-Br-$C_6H_4$ | $CH_3$ | $SCH_3$ | 3 | N | | |
| 160 | 4-Br-$C_6H_4$ | $CH_3$ | $OCH_3$ | 4 | N | | |
| 161 | 4-Br-$C_6H_4$ | $CH_3$ | $OCH_3$ | 4 | CH | | |
| 162 | 4-Br-$C_6H_4$ | $CH_3$ | $OCH_3$ | 5 | N | | |
| 163 | 4-Br-$C_6H_4$ | $CH_3$ | $OCH_3$ | 5 | CH | | |
| 164 | 2-F-$C_6H_4$ | $CH_3$ | $OCH_3$ | 0 | N | | |
| 165 | 2-F-$C_6H_4$ | $CH_3$ | $OCH_3$ | 0 | CH | | |
| 166 | 2-F-$C_6H_4$ | $CH_3$ | $OC_2H_5$ | 0 | N | | |

Tabelle (Fortsetzung)

| Bsp | A | B | D | n | X | Schmp./IR ($cm^{-1}$) | Isomer* |
|---|---|---|---|---|---|---|---|
| 167 | 2-F-$C_6H_4$ | $CH_3$ | $OCH_3$ | 1 | N | | |
| 168 | 2-F-$C_6H_4$ | $CH_3$ | $SCH_3$ | 1 | N | | |
| 169 | 2-F-$C_6H_4$ | $CH_3$ | $OCH_3$ | 2 | N | | |
| 170 | 2-F-$C_6H_4$ | $CH_3$ | $OCH_3$ | 3 | N | | |
| 171 | 2-F-$C_6H_4$ | $CH_3$ | $OCH_3$ | 4 | N | | |
| 172 | 2-Cl-$C_6H_4$ | $CH_3$ | $OCH_3$ | 0 | N | | |
| 173 | 2-Cl-$C_6H_4$ | $CH_3$ | $OCH_3$ | 1 | N | | |
| 174 | 2-Cl-$C_6H_4$ | $CH_3$ | $OCH_3$ | 1 | CH | | |
| 175 | 2-Cl-$C_6H_4$ | $CH_3$ | $OCH_3$ | 2 | N | | |
| 176 | 2-Cl-$C_6H_4$ | $CH_3$ | $OCH_3$ | 3 | N | | |
| 177 | 2-Cl-$C_6H_4$ | $CH_3$ | $OCH_3$ | 4 | N | | |
| 178 | 2-Cl-$C_6H_4$ | $CH_3$ | $OCH_3$ | 5 | N | | |
| 179 | 4-$OCH_3$-$C_6H_4$ | $CH_3$ | $OCH_3$ | 0 | N | | |
| 180 | 4-$OCH_3$-$C_6H_4$ | $CH_3$ | $OCH_3$ | 1 | N | | |
| 181 | 4-$OCH_3$-$C_6H_4$ | $CH_3$ | $SCH_3$ | 1 | N | | |
| 182 | 4-$OCH_3$-$C_6H_4$ | $CH_3$ | 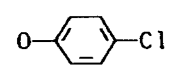 | 1 | N | | |
| 183 | 4-$OCH_3$-$C_6H_4$ | $CH_3$ | $OCH_3$ | 2 | N | | |
| 184 | 4-$OCH_3$-$C_6H_4$ | $CH_3$ | $OCH_3$ | 3 | N | | |
| 185 | 4-$CH_3$-$C_6H_4$ | $CH_3$ | $OCH_3$ | 0 | N | | |
| 186 | 4-$CH_3$-$C_6H_4$ | $CH_3$ | $OCH_3$ | 1 | N | | |
| 187 | 4-$CH_3$-$C_6H_4$ | $CH_3$ | $SCH_3$ | 1 | N | | |
| 188 | 4-$CH_3$-$C_6H_4$ | $CH_3$ | 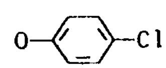 | 1 | N | | |

Tabelle (Fortsetzung)

| Bsp | A | B | D | n | X | Schmp./IR ($cm^{-1}$) | Isomer* |
|---|---|---|---|---|---|---|---|
| 189 | 4-$CH_3$-$C_6H_4$ | $CH_3$ | $OCH_3$ | 3 | N | | |
| 190 | 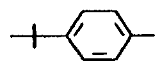 | $CH_3$ | $OCH_3$ | 1 | N | | |
| 191 | 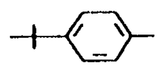 | $CH_3$ | $OCH_3$ | 2 | N | | |
| 192 | 1-Naphthyl | $CH_3$ | $OCH_3$ | 0 | N | | |
| 193 | 1-Naphthyl | $CH_3$ | $OCH_3$ | 1 | N | | |
| 194 | 1-Naphthyl | $CH_3$ | $OCH_3$ | 2 | N | | |
| 195 | 1-Naphthyl | $CH_3$ | $SCH_3$ | 2 | N | | |
| 196 | 1-Naphthyl | $CH_3$ | 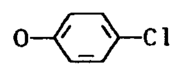 | 0 | N | | |
| 197 | 2-Naphthyl | $CH_3$ | $OCH_3$ | 0 | N | | |
| 198 | 2-Naphthyl | $CH_3$ | $OCH_3$ | 1 | N | | |
| 199 | 2-Naphthyl | $CH_3$ | $SCH_3$ | 1 | N | | |
| 200 | 1-Naphthyl | $CH_3$ | 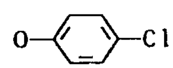 | 1 | N | | |
| 201 | 2-Naphthyl | $CH_3$ | $OCH_3$ | 2 | N | | |
| 202 | 2-Naphthyl | $CH_3$ | $OCH_3$ | 3 | N | | |
| 203 | 2-Naphthyl | $CH_3$ | $OCH_3$ | 4 | N | | |
| 204 | 2-Naphthyl | $CH_3$ | $OCH_3$ | 5 | N | | |

Tabelle (Fortsetzung)

| Bsp | A | B | D | n | X | Schmp./IR ($cm^{-1}$) | Isomer* |
|---|---|---|---|---|---|---|---|
| 205 | tert.-$C_4H_9$ | 4-F-$C_6H_4$ | $OCH_3$ | 0 | N | | |
| 206 | tert.-$C_4H_9$ | 4-F-$C_6H_4$ | $OC_2H_5$ | 0 | N | | |
| 207 | tert.-$C_4H_9$ | 4-F-$C_6H_4$ | $SCH_3$ | 0 | N | | |
| 208 | tert.-$C_4H_9$ | 4-F-$C_6H_4$ | 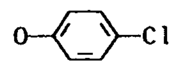 | 0 | N | | |
| 209 | tert.-$C_4H_9$ | 4-Cl-$C_6H_4$ | $OCH_3$ | 0 | N | | |
| 210 | tert.-$C_4H_9$ | 4-Cl-$C_6H_4$ | $OC_2H_5$ | 1 | N | | |
| 211 | tert.-$C_4H_9$ | 4-Cl-$C_6H_4$ | $SCH_3$ | 0 | N | | |
| 212 | tert.-$C_4H_9$ | 2-Cl-$C_6H_4$ | $OCH_3$ | 0 | N | | |
| 213 | tert.-$C_4H_9$ | 2-Cl-$C_6H_4$ | $OC_2H_4$ | 0 | N | | |
| 214 | tert.-$C_4H_9$ | 2-Cl-$C_6H_4$ | 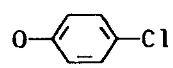 | 0 | N | | |
| 215 | tert.-$C_4H_9$ | 2,4-$Cl_2$-$C_6H_3$ | $OCH_3$ | 0 | N | | |
| 216 | tert.-$C_4H_9$ | 2,4-$Cl_2$-$C_6H_3$ | $OCH_3$ | 1 | N | | |
| 217 | tert.-$C_4H_9$ | 2,4-$Cl_2$-$C_6H_3$ | $OCH_3$ | 2 | N | | |
| 218 | tert.-$C_4H_9$ | 2,4-$Cl_2$-$C_6H_3$ | $OC_2H_5$ | 1 | N | | |
| 219 | tert.-$C_4H_9$ | 4-$OCH_3$-$C_6H_4$ | $OCH_3$ | 0 | N | | |
| 220 | tert.-$C_4H_9$ | 4-$CH_3$-$C_6H_4$ | $OCH_3$ | 1 | N | | |
| 221 | tert.-$C_4H_9$ | 4-$NO_2$-$C_6H_4$ | $OCH_3$ | 1 | N | | |
| 222 | tert.-$C_4H_9$ | Cyclohexyl | $OCH_3$ | 0 | N | | |
| 223 | tert.-$C_4H_9$ | Cyclohexyl | $OC_2H_5$ | 0 | N | | |
| 224 | tert.-$C_4H_9$ | Cyclohexyl | $OCH_3$ | 1 | N | | |

Tabelle (Fortsetzung)

| Bsp | A | B | D | n | X | Schmp./IR ($cm^{-1}$) | Isomer* |
|---|---|---|---|---|---|---|---|
| 225 | 2,4-Cl-$C_6H_3$ | $CH_3$ | S-$C_6H_5$ | 2 | N | 2959, 1474, 1273, 1136, 822 | $D_1$:$D_2$ = 2:1 |
| 226 | $C_6H_5$ | $CH_3$ | S-$C_6H_5$ | 1 | N | 2965, 1499, 1274, 1136, 744, 700 | $D_1$:$D_2$ = 2:1 |
| 227 | $C_6H_5$ | $CH_3$ | 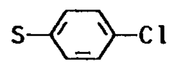 | 1 | N | 2969, 1498, 1476, 1274, 1136, 1094, 1013, 823, 700 | $D_1$:$D_2$ = 2:1 |
| 228 | 2,4-$Cl_2$-$C_6H_3$ | $CH_3$ | S-$C_6H_5$ | 4 | N | 2955, 2929, 1501, 1474, 1273, 1136, 742, 691 | $D_1$:$D_2$ = 1:1 |
| 229 | $C_6H_5$ | 2-F-$C_6H_4$ | S-$C_6H_5$ | 0 | N | 1491, 1480, 1453, 1275, 757, 739, 697 | $D_1$:$D_2$ = 1:1 |
| 230 | $C_6H_5$ | $CH_3$ | 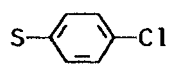 | 3 | N | 2955, 2930, 1476, 1095, 1013, 812, 700 | $D_1$:$D_2$ = 1:1 |
| 231 | $C_6H_5$ | $CH_3$ | 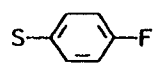 | 3 | N | 2956, 1490, 1229, 820, 700 | $D_1$:$D_2$ = 1:1 |
| 232 | 4-Cl-$C_6H_4$ | CH§ | S-$C_6H_5$ | 0 | N | 1598, 1476, 1273, 1137, 742, 690 | $D_1$:$D_2$ = 2:1 |
| 233 | 4-Cl-$C_6H_4$ | $CH_3$ | S-$C_6H_5$ | 0 | N | 108-110°C | Enantiomerengemisch |

*Verhältnis der gebildeten Diastereomeren

Tabelle (Fortsetzung)

| Bsp | A | B | D | n | X | Schmp./IR ($cm^{-1}$) | Isomer* |
|---|---|---|---|---|---|---|---|
| 234 | $C_6H_5$ | $C_6H_5$ | $OCH_3$ | 0 | N | | |
| 235 | $C_6H_5$ | $C_6H_5$ | $OCH_3$ | 0 | CH | | |
| 236 | $C_6H_5$ | $C_6H_5$ | $OC_2H_5$ | 0 | N | | |
| 237 | $C_6H_5$ | $2-Cl-C_6H_4$ | $OCH_3$ | 0 | N | | |
| 238 | $C_6H_5$ | $4-CL-C_6H_4$ | $OCH_3$ | 0 | N | | |
| 239 | $C_6H_5$ | $2,4-Cl_2-C_6H_3$ | $OCH_3$ | 0 | N | | |
| 240 | $C_6H_5$ | $2-F-C_6H_4$ | $OCH_3$ | 0 | N | | |
| 241 | $C_6H_5$ | $4-F-C_6H_4$ | $OCH_3$ | 0 | N | | |
| 242 | $C_6H_5$ | $4-F-C_6H_4$ | $OC_2H_5$ | 0 | N | | |
| 243 | $C_6H_5$ | $2-CH_3-C_6H_4$ | $OCH_3$ | 0 | N | | |
| 244 | $C_6H_5$ | $2,4-(CH_3)_2-C_6H_3$ | $OCH_3$ | 0 | N | | |
| 245 | $C_6H_5$ | $4-CF_3-C_6H_4$ | $OCH_3$ | 0 | N | | |
| 246 | $C_6H_5$ | $4-Br-C_6H_4$ | $OCH_3$ | 0 | N | | |
| 247 | $C_6H_5$ | $4-OCH_3-C_6H_4$ | $OCH_3$ | 0 | N | | |
| 248 | $C_6H_5$ | $4-CH_3-C_6H_4$ | $OCH_3$ | 0 | N | | |
| 249 | $C_6H_5$ | 2-Naphtyl | $OCH_3$ | 0 | N | | |
| 250 | $4-F-C_6H_4$ | $2-F-C_6H_4$ | $OCH_3$ | 0 | N | | |
| 251 | $4-F-C_6H_4$ | $2-F-C_6H_4$ | $OCH_3$ | 0 | CH | | |
| 252 | $4-F-C_6H_4$ | $2-F-C_6H_4$ | $OC_2H_5$ | 0 | N | | |
| 253 | $4-F-C_6H_4$ | $2-F-C_6H_4$ | $OCH(CH_3)_2$ | 0 | N | | |
| 254 | $4-F-C_6H_4$ | $4-F-C_6H_4$ | $OCH_3$ | 0 | N | | |
| 255 | $4-F-C_6H_4$ | $4-F-C_6H_4$ | $OCH_3$ | 0 | CH | | |
| 256 | $4-F-C_6H_4$ | $4-F-C_6H_4$ | $OC_2H_5$ | 0 | N | | |

Tabelle (Fortsetzung)

| Bsp | A | B | D | n | X | Schmp./IR ($cm^{-1}$) | Isomer* |
|---|---|---|---|---|---|---|---|
| 257 | 4-F-$C_6H_4$ | 4-F-$C_6H_4$ | $OC_2H_5$ | 0 | CH | | |
| 258 | 4-F-$C_6H_4$ | 4-F-$C_6H_4$ | $OCH(CH_3)_2$ | 0 | N | | |
| 259 | 4-F-$C_6H_4$ | 4-F-$C_6H_4$ | $OCH_2C_6H_5$ | 0 | N | | |
| 260 | 4-F-$C_6H_4$ | 2-Cl-$C_6H_4$ | $OCH_3$ | 0 | N | | |
| 261 | 4-F-$C_6H_4$ | 2-Cl-$C_6H_4$ | $OCH_3$ | 0 | CH | | |
| 262 | 4-F-$C_6H_4$ | 2-Cl-$C_6H_4$ | $OC_2H_5$ | 0 | N | | |
| 263 | 4-F-$C_6H_4$ | 2-Cl-$C_6H_4$ | $OC_2H_5$ | 0 | CH | | |
| 264 | 4-F-$C_6H_4$ | 2-Cl-$C_6H_4$ | $OCH(CH_3)_2$ | 0 | N | | |
| 265 | 4-F-$C_6H_4$ | 2-Cl-$C_6H_4$ | $OCH(CH_3)_2$ | 0 | CH | | |
| 266 | 4-F-$C_6H_4$ | 4-Cl-$C_6H_4$ | $OCH_3$ | 0 | N | | |
| 267 | 4-F-$C_6H_4$ | 4-Cl-$C_6H_4$ | $OCH_3$ | 0 | CH | | |
| 268 | 4-F-$C_6H_4$ | 4-Cl-$C_6H_4$ | $OC_2H_5$ | 0 | N | | |
| 269 | 4-F-$C_6H_4$ | 4-Cl-$C_6H_4$ | $OC_2H_5$ | 0 | CH | | |
| 270 | 4-F-$C_6H_4$ | 4-Cl-$C_6H_4$ | $OCH(CH_3)_2$ | 0 | N | | |
| 271 | 4-F-$C_6H_4$ | 2,4-$Cl_2$-$C_6H_3$ | $OCH_3$ | 0 | N | | |
| 272 | 4-F-$C_6H_4$ | 2,4-$Cl_2$-$C_6H_3$ | $OCH_3$ | 0 | CH | | |
| 273 | 4-F-$C_6H_4$ | 2,4-$Cl_2$-$C_6H_3$ | $OC_2H_5$ | 0 | N | | |
| 274 | 4-F-$C_6H_4$ | 2,4-$Cl_2$-$C_6H_3$ | $OC_2H_5$ | 0 | N | | |
| 275 | 4-F-$C_6H_4$ | 2,4-$Cl_2$-$C_6H_3$ | $OCH(CH_3)_2$ | 0 | N | | |
| 276 | 4-F-$C_6H_4$ | 4-Br-$C_6H_4$ | $OCH_3$ | 0 | N | | |
| 277 | 4-F-$C_6H_4$ | 4-Br-$C_6H_4$ | $OC_2H_5$ | 0 | N | | |
| 278 | 4-F-$C_6H_4$ | 2-$CH_3$-$C_6H_4$ | $OCH_3$ | 0 | N | | |
| 279 | 4-F-$C_6H_4$ | 2-$CH_3$-$C_6H_4$ | $OCH_3$ | 0 | CH | | |

Tabelle (Fortsetzung)

| Bsp | A | B | D | n | X | Schmp./IR ($cm^{-1}$) | Isomer* |
|---|---|---|---|---|---|---|---|
| 280 | 4-F-$C_6H_4$ | 2-$CH_3$-$C_6H_4$ | $OC_2H_5$ | 0 | N | | |
| 281 | 4-F-$C_6H_4$ | 2-$CH_3$-$C_6H_4$ | $OCH(CH_3)_2$ | 0 | N | | |
| 282 | 4-F-$C_6H_4$ | 4-$CH_3$-$C_6H_4$ | $OCH_3$ | 0 | N | | |
| 283 | 4-F-$C_6H_4$ | 4-$CH_3$-$C_6H_4$ | $OCH_3$ | 0 | CH | | |
| 284 | 4-F-$C_6H_4$ | 4-$CH_3$-$C_6H_4$ | $OC_2H_5$ | 0 | N | | |
| 285 | 4-F-$C_6H_4$ | 4-$CH_3$-$C_6H_4$ | $OCH(CH_3)_2$ | 0 | N | | |
| 286 | 4-F-$C_6H_4$ | 2,4-$(CH_3)_2$-$C_6H_3$ | $OCH_3$ | 0 | N | | |
| 287 | 4-F-$C_6H_4$ | 2,4-$(CH_3)_2$-$C_6H_3$ | $OC_2H_5$ | 0 | N | | |
| 288 | 4-F-$C_6H_4$ | 4-$CF_3$-$C_6H_4$ | $OCH_3$ | 0 | N | | |
| 289 | 4-F-$C_6H_4$ | 4-$CF_3$-$C_6H_4$ | $OC_2H_5$ | 0 | N | | |
| 290 | 4-F-$C_6H_4$ | 4-$OCH_3$-$C_6H_4$ | $OCH_3$ | 0 | N | | |
| 291 | 4-F-$C_6H_4$ | 4-$OCH_3$-$C_6H_4$ | $OC_2H_5$ | 0 | N | | |
| 292 | 4-Cl-$C_6H_4$ | 2-F-$C_6H_4$ | $OCH_3$ | 0 | N | | |
| 293 | 4-Cl-$C_6H_4$ | 2-F-$C_6H_4$ | $OC_2H_5$ | 0 | N | | |
| 294 | 4-Cl-$C_6H_4$ | 2-Cl-$C_6H_4$ | $OCH_3$ | 0 | N | | |
| 295 | 4-Cl-$C_6H_4$ | 2-Cl-$C_6H_4$ | $OC_2H_5$ | 0 | N | | |
| 296 | 4-Cl-$C_6H_4$ | 4-Cl-$C_6H_4$ | $OCH_3$ | 0 | N | | |
| 297 | 4-Cl-$C_6H_4$ | 4-Cl-$C_6H_4$ | $OCH_3$ | 0 | CH | | |
| 298 | 4-Cl-$C_6H_4$ | 4-Cl-$C_6H_4$ | $OC_2H_5$ | 0 | N | | |
| 299 | 4-Cl-$C_6H_4$ | 4-Cl-$C_6H_4$ | $OC_2H_5$ | 0 | CH | | |
| 300 | 4-Cl-$C_6H_4$ | 4-Cl-$C_6H_4$ | $OCH(CH_3)_2$ | 0 | N | | |
| 301 | 4-Cl-$C_6H_4$ | 4-Cl-$C_6H_4$ | $OCH(CH_3)_2$ | 0 | CH | | |
| 302 | 4-Cl-$C_6H_4$ | 2,4-Cl-$C_6H_3$ | $OCH_3$ | 0 | N | | |

Tabelle (Fortsetzung)

| Bsp | A | B | D | n | X | Schmp./IR ($cm^{-1}$) | Isomer* |
|---|---|---|---|---|---|---|---|
| 303 | 4-Cl-$C_6H_4$ | 2,4-Cl-$C_6H_3$ | $OC_2H_5$ | 0 | N | | |
| 304 | 4-Cl-$C_6H_4$ | 2-$CH_3$-$C_6H_4$ | $OCH_3$ | 0 | N | | |
| 305 | 4-Cl-$C_6H_4$ | 2-$CH_3$-$C_6H_4$ | $OC_2H_5$ | 0 | N | | |
| 306 | 4-Cl-$C_6H_4$ | 2,4-$(CH_3)_2$-$C_6H_3$ | $OCH_3$ | 0 | N | | |
| 307 | 4-Cl-$C_6H_4$ | 2,4-$(CH_3)_2$-$C_6H_3$ | $OC_2H_5$ | 0 | N | | |
| 308 | 4-Cl-$C_6H_4$ | 4-$CF_3$-$C_6H_4$ | $OCH_3$ | 0 | N | | |
| 309 | 4-Cl-$C_6H_4$ | 4-$CF_3$-$C_6H_4$ | $OC_2H_5$ | 0 | N | | |
| 310 | 4-Cl-$C_6H_4$ | 4-Br-$C_6H_4$ | $OCH_3$ | 0 | N | | |
| 311 | 4-Cl-$C_6H_4$ | 4-Br-$C_6H_4$ | $OC_2H_5$ | 0 | N | | |
| 312 | 4-Cl-$C_6H_4$ | 4-$CH_3$-$C_6H_4$ | $OCH_3$ | 0 | N | | |
| 313 | 4-Cl-$C_6H_4$ | 4-$CH_3$-$C_6H_4$ | $OCH_3$ | 0 | CH | | |
| 314 | 4-Cl-$C_6H_4$ | 4-$CH_3$-$C_6H_4$ | $OC_2H_5$ | 0 | N | | |
| 315 | 4-$CH_3$-$C_6H_4$ | 2-F-$C_6H_4$ | $OCH_3$ | 0 | N | | |
| 316 | 4-$CH_3$-$C_6H_4$ | 2-Cl-$C_6H_4$ | $OCH_3$ | 0 | N | | |
| 317 | 4-$CH_3$-$C_6H_4$ | 2,4-$Cl_2$-$C_6H_3$ | $OCH_3$ | 0 | N | | |
| 318 | 4-$CH_3$-$C_6H_4$ | 4-Br-$C_6H_4$ | $OCH_3$ | 0 | N | | |
| 319 | 4-$CH_3$-$C_6H_4$ | 4-$CF_3$-$C_6H_4$ | $OCH_3$ | 0 | N | | |
| 320 | 4-$CH_3$-$C_6H_4$ | 2-$CH_3$-$C_6H_4$ | $OCH_3$ | 0 | N | | |
| 321 | 4-$CH_3$-$C_6H_4$ | 4-$CH_3$-$C_6H_4$ | $OCH_3$ | 0 | N | | |
| 322 | 4-$CH_3$-$C_6H_4$ | 4-$CH_3$-$C_6H_4$ | $OCH_3$ | 0 | CH | | |
| 323 | 4-$CH_3$-$C_6H_4$ | 4-$CH_3$-$C_6H_4$ | $OC_2H_5$ | 0 | N | | |
| 324 | 4-$CH_3$-$C_6H_4$ | 4-$CH_3$-$C_6H_4$ | $OC_2H_5$ | 0 | CH | | |
| 325 | 4-$CH_3$-$C_6H_4$ | 4-$CH_3$-$C_6H_4$ | $OCH(CH_3)_2$ | 0 | N | | |
| 326 | 4-$CH_3$-$C_6H_4$ | 4-$OCH_3$-$C_6H_4$ | $OCH_3$ | 0 | N | | |

Tabelle (Fortsetzung)

| Bsp | A | B | D | n | X | Schmp./IR ($cm^{-1}$) | Isomer* |
|---|---|---|---|---|---|---|---|
| 327 | $4-CH_3-C_6H_4$ | $4-OCH_3-C_6H_4$ | $OC_2H_5$ | 0 | N | | |
| 328 | $4-OCH_3-C_6H_4$ | $2-F-C_6H_4$ | $OCH_3$ | 0 | N | | |
| 329 | $4-OCH_3-C_6H_4$ | $2-Cl-C_6H_4$ | $OCH_3$ | 0 | N | | |
| 330 | $4-OCH_3-C_6H_4$ | $2,4-Cl_2-C_6H_3$ | $OCH_3$ | 0 | N | | |
| 331 | $4-OCH_3-C_6H_4$ | $4-Br-C_6H_4$ | $OCH_3$ | 0 | N | | |
| 332 | $4-OCH_3-C_6H_4$ | $4-CF-C_6H_4$ | $OCH_3$ | 0 | N | | |
| 333 | $4-OCH_3-C_6H_4$ | $2-CH_3-C_6H_4$ | $OCH_3$ | 0 | N | | |
| 334 | $4-OCH_3-C_6H_4$ | $2,4-(CH_3)_2-C_6H_3$ | $OCH_3$ | 0 | N | | |
| 335 | $4-OCH_3-C_6H_4$ | $4-OCH_3-C_6H_4$ | $OCH_3$ | 0 | N | | |
| 336 | $4-OCH_3-C_6H_4$ | $4-OCH_3-C_6H_4$ | $OCH_3$ | 0 | CH | | |
| 337 | $4-OCH_3-C_6H_4$ | $4-OCH_3-C_6H_4$ | $OC_2H_5$ | 0 | N | | |
| 338 | $4-OCH_3-C_6H_4$ | $4-OCH_3-C_6H_4$ | $OC_2H_5$ | 0 | CH | | |
| 339 | $4-OCH_3-C_6H_4$ | $4-OCH_3-C_6H_4$ | $OCH(CH_3)_2$ | 0 | N | | |
| 340 | $4-CF_3-C_6H_4$ | $2-F-C_6H_4$ | $OCH_3$ | 0 | N | | |
| 341 | $4-CF_3-C_6H_4$ | $2-Cl-C_6H_4$ | $OCH_3$ | 0 | N | | |
| 342 | $4-CF_3-C_6H_4$ | $2-CH_3-C_6H_4$ | $OCH_3$ | 0 | N | | |
| 343 | $4-CF_3-C_6H_4$ | $2,4-(CH_3)_2-C_6H_4$ | $OCH_3$ | 0 | N | | |
| 344 | $4-CF_3-C_6H_4$ | $4-Br-C_6H_4$ | $OCH_3$ | 0 | N | | |
| 345 | $4-CF_3-C_6H_4$ | $4-CF_3-C_6H_4$ | $OCH_3$ | 0 | N | | |
| 346 | $4-CF_3-C_6H_4$ | $4-CF_3-C_6H_4$ | $OCH_3$ | 0 | CH | | |
| 347 | $4-CF_3-C_6H_4$ | $4-CF_3-C_6H_4$ | $OC_2H_5$ | 0 | N | | |
| 348 | $4-CF_3-C_6H_4$ | $4-CF_3-C_6H_4$ | $OC_2H_5$ | 0 | CH | | |
| 349 | $4-CF_3-C_6H_4$ | $4-CF_3-C_6H_4$ | $OCH(CH_3)_2$ | 0 | N | | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,

Erysiphe cichoracearum und Sphaerotheca fuliginea an Kürbisgewächsen,

Podosphaera leucotricha an Äpfeln,

Uncinula necator an Reben,

Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Verbindungen können praktisch alle Entwicklungstadien einer Pflanze verschiedenartig beeinflussen und werden deshalb als Wachstumsregulatoren eingesetzt. Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze und von der Jahreszeit,

c) von dem Applikationsort und -verfahren (Samenbeize, Bodenbehandlung oder Blattapplikation)

d) von klimatischen Faktoren, z.B. Temperatur, Niederschlagsmenge, außerdem auch Tageslänge und Lichtintensität

e) von der Bodenbeschaffenheit (einschließlich Düngung),

f) von der Formulierung bzw. Anwendungsform des Wirkstoffs und schließlich

g) von den angewendeten Konzentrationen der aktiven Substanz.

Aus der Reihe der verschiedenartigen Anwendungsmöglichkeiten der erfindungsgemäßen Pflanzenwachstumsregulatoren im Pflanzenanbau, werden einige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist sich z.B. die Verringerung des Grasbewuchses an Straßenrändern, Hecken, Kanalböschungen und auf Rasenflächen wie Park-, Sport- und Obstanlagen, Zierrasen und Flugplätzen, so daß der arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Reis, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt.

Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- und Pflanzenmasse - dem Befall mit verschiedenen

Krankheiten (z.B. Pilzkrankheit) vorgebeugt werden. Die Hemmung des vegetativen Wachstums ermöglicht außerdem bei vielen Kulturpflanzen die dichtere Bepflanzung des Bodens, so daß ein Mehrertrag, bezogen auf die Bodenfläche, erzielt werden kann.

B. Mit den neuen Mitteln lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die Verbindungen der Formel I Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftichem Interesse ist beispielsweise die Ernteerleicherung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wesentlich.

D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite des Stomata reduziert wird
- eine dickere Epidermis und Cuticula ausgebildet werden
- die Durchwurzelung des Bodens verbessert wird
- das Mikroklima im Pflanzenbestand durch einen kompakteren Wuchs günstig beeinflußt wird.

Die erfindungsgemäß zu verwendeten Wirkstoffe können den Kulturpflanzen sowohl vom Samen her (als Saatgutbeizmittel) als auch über den Boden, d.h. durch die Wurzel sowie über das Blatt zugeführt werden.

Infolge der hohen Pflanzenverträglichkeit kann die Aufwandmenge als Wachstumsregulatoren stark variiert werden.

Bei der Saatgutbehandlung werden im allgemeinen Wirkstoffmengen von 0,001 bis 50 g, vorzugsweise 0,01 bis 10 g je Kilogramm Saatgut benötigt.

Für die Blatt- und Bodenbehandlung sind im allgemeinen Gaben von 0,01 bis 10 kg/ha, bevorzugt 0,02 bis 3 kg/ha, als ausreichend zu betrachten.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zübereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 1 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 27 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzol sulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 42 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 44 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 1 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 27 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 42 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 44 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 1 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiele

Als Vergleichswirkstoffe wurden die Verbindungen 1-(1,2,4-Triazol-1-yl)-1-phenylthio-2-methyl-2-phenylpropan (A) und 1-(1,2,4-Triazol-1-yl)-1-(4-chlorphenylthio)-2-methyl-2-phenylpropan (B) - bekannt aus EP 91219 - verwendet.

Anwendungsbeispiel 1

Wirksamkeit gegen Pyricularia oryzae (protektiv)

Blätter von in Töpfen gewachsenen Reiskeimlingen der Sorte "Bahia" wurden mit wäßrigen Emulsionen, die 80 % Wirkstoff und 20 % Emulgiermittel in der Trockensubstanz enthielten, tropfnaß besprüht und 24 Stunden später mit einer wäßrigen Sporensuspension von Pyricularia oryzae inokuliert. Anschließend wurden die Versuchspflanzen in Klimakammern bei 22 bis 24 °C und 95 bis 99 % relativer Luftfeuchtigkeit aufgestellt. Nach 6 Tagen wurde das Ausmaß des Krankheitsbefalls ermittelt.

Das Ergebnis zeigt, daß die Wirkstoffe 1, 27, 42, 44, 228 und 232 bei der Anwendung als 0,05 %ige

(Gew.%) Spritzbrühe eine bessere fungizide Wirkung zeigen (90 %) als die bekannten Vergleichswirkstoffe A und B (30 %).

Anwendungsbeispiel 2

Wirksamkeit gegen Pyrenophora teres

Gerstenkeimlinge der Sorte "Igri" wurden im Zweiblattstadium mit wäßrigen Suspensionen, die 80 % Wirkstoff und 20 % Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunden ineine Klimakammer mit hoher Luftfeuchtigkeit bei 18°C gestellt. Anschließend wurden die Pflanzen im Gewächshaus bei 20 bis 22°C und 70 % relativer Luftfeuchtigkeit für weitere 5 Tage kultiviert. Dann wurde das Ausmaß des Blattbefalls ermittelt.

Das Ergebnis zeigt, daß die Wirkstoffe 1, 27, 49, 134, 226 und 228 bei der Anwendung als 0,05 %ige Spritzbrühe eine bessere fungizide Wirkung zeigen (90 %) als die bekannten Vergleichswirkstoffe A und B (10 %).

## Ansprüche

1. Azolylethanderivate der allgemeinen Formel I

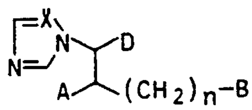

I

in welcher
A und B $C_1$-$C_8$-Alkyl, Phenyl, Biphenyl, Naphthyl, Benzyl, Tetrahydropyranyl, $C_3$-$C_8$-Cycloalkyl oder $C_5$-$C_8$-Cycloalkenyl bedeuten,
wobei diese Reste einfach bis dreifach durch Halogen, Nitro, Phenoxy, Amino, Alkyl, Alkoxy oder Halogenalkyl mit jeweils 1-bis 4-C-Atomen substituiert sein können,
D $C_1$-$C_4$-Alkoxy, Amino, gegebenenfalls durch $C_1$-$C_3$-Alkyl, $C_1$-$C_3$-Acyl substituiertes Amino, gegebenenfalls durch $C_1$-$C_4$-Alkyl substituiertes Mercapto, Thiophenyl, Aminophenyl, Hetaryloxy oder Phenoxy bedeutet,
wobei diese Reste einfach bis dreifach durch Halogen, Nitro, Phenoxy, Amino, Alkyl oder Halogenalkyl mit jeweils 1 bis 4 C-Atomen substituiert sein können,
n eine ganze Zahl von 1 bis 5 oder 0 bedeutet,
X den Rest CH oder N bedeutet,
sowie deren für Pflanzen verträgliche Säureadditionssalze und Metallkomplexe, außer den Verbindungen, in denen A unsubstituiertes Phenyl, B Alkyl, D Alkoxy, X N und n 0 bedeuten.

2. Verfahren zur Herstellung der Azolylethanderivate der Formel I gemäß Anspruch 1, dadurch gekennzeichnet, daß man eine Verbindung der Formel II

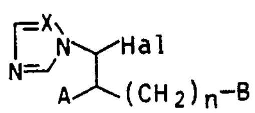

II

in welcher
A, B und X die im Anspruch 1 genannte Bedeutung haben und Hal Chlor oder Brom bedeutet,
mit einer Verbindung der Formel D-H umsetzt und die so erhaltenen Verbindungen gegebenenfalls in ihre für Pflanzen verträglichen Säureadditionssalze oder Metallkomplexe überführt.

3. Fungizides Mittel, enthaltend einen Trägerstoff und eine fungizid wirksame Menge eines Azolylethanderivats der allgemeinen Formel I

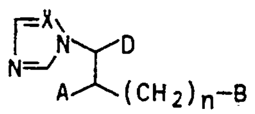

I

in welcher
A und B $C_1$-$C_8$-Alkyl, Phenyl, Biphenyl, Naphthyl, Benzyl, Tetrahydropyranyl, $C_3$-$C_8$-Cycloalkyl oder $C_5$-$C_8$-Cycloalkenyl bedeuten,
wobei diese Reste einfach bis dreifach durch Halogen, Nitro, Phenoxy, Amino, Alkyl, Alkoxy oder Halogenalkyl mit jeweils 1-bis 4-C-Atomen substituiert sein können,
D $C_1$-$C_4$-Alkoxy, Amino, gegebenenfalls durch $C_1$-$C_3$-Alkyl, $C_1$-$C_3$-Acyl substituiertes Amino, gegebenenfalls durch $C_1$-$C_4$-Alkyl substituiertes Mercapto, Thiophenyl, Aminophenyl, Hetaryloxy oder Phenoxy bedeutet,
wobei diese Reste einfach bis dreifach durch Halogen, Nitro, Phenoxy, Amino, Alkyl oder Halogenalkyl mit jeweils 1 bis 4 C-Atomen substituiert sein können,
n eine ganze Zahl von 1 bis 5 oder 0 bedeutet,
X den Rest CH oder N bedeutet,
oder dessen für Pflanzen verträgliches Säureadditionssalz oder Metallkomplex, außer den Verbindungen, in denen A unsubstituiertes Phenyl, B Alkyl, D Alkoxy, X N und n 0 bedeuten.

4. Verfahren zur Bekämpfung von Pilzen, dadurch gekennzeichnet, daß man eine fungizid wirksame Menge eines Azolylethanderivates der Formel I

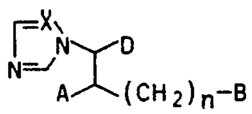

I

in welcher
A und B $C_1$-$C_8$-Alkyl, Phenyl, Biphenyl, Naphthyl, Benzyl, Tetrahydropyranyl, $C_3$-$C_8$-Cycloalkyl oder $C_5$-$C_8$-Cycloalkenyl bedeuten,
wobei diese Reste einfach bis dreifach durch Halogen, Nitro, Phenoxy, Amino, Alkyl, Alkoxy oder Halogenalkyl mit jeweils 1-bis 4-C-Atomen substituiert sein können,
D $C_1$-$C_4$-Alkoxy, Amino, gegebenenfalls durch $C_1$-$C_3$-Alkyl, $C_1$-$C_3$-Acyl substituiertes Amino, gegebenenfalls durch $C_1$-$C_4$-Alkyl substituiertes Mercapto, Thiophenyl, Aminophenyl, Hetaryloxy oder Phenoxy bedeutet,
wobei diese Reste einfach bis dreifach durch Halogen, Nitro, Phenoxy, Amino, Alkyl oder Halogenalkyl mit jeweils 1 bis 4 C-Atomen substituiert sein können,
n eine ganze Zahl von 1 bis 5 oder 0 bedeutet,
X den Rest CH oder N bedeutet,
oder dessen für Pflanzen verträgliche Säureadditionssalz und Metallkomplex, außer den Verbindungen, in denen A unsubstituiertes Phenyl, B Alkyl, D Alkoxy, X N und n 0 bedeuten, auf die Pilze oder durch Pilzbefall bedrohte Materialien, Flächen, Pflanzen und Saatgüter einwirken läßt.

5. Wachstumsregulatorisches Mittel, enthaltend einen Trägerstoff und eine regulierend wirksame Menge eines Azolylethanderivats der Formel I

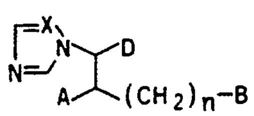

I

in welcher
A und B $C_1$-$C_8$-Alkyl, Phenyl, Biphenyl, Naphthyl, Benzyl, Tetrahydropyranyl, $C_3$-$C_8$-Cycloalkyl oder $C_5$-$C_8$-Cycloalkenyl bedeuten,
wobei diese Reste einfach bis dreifach durch Halogen, Nitro, Phenoxy, Amino, Alkyl, Alkoxy oder Halogenalkyl mit jeweils 1-bis 4-C-Atomen substituiert sein können,
D $C_1$-$C_4$-Alkoxy, Amino, gegebenenfalls durch $C_1$-$C_3$-Alkyl, $C_1$-$C_3$-Acyl substituiertes Amino, gegebenenfalls durch $C_1$-$C_4$-Alkyl substituiertes Mercapto, Thiophenyl, Aminophenyl, Hetaryloxy oder Phenoxy bedeutet,
wobei diese Reste einfach bis dreifach durch Halogen, Nitro, Phenoxy, Amino, Alkyl oder Halogenalkyl mit jeweils 1-bis 4-C-Atomen substituiert sein können,
n eine ganze Zahl von 1 bis 5 oder 0 bedeutet,
X den Rest CH oder N bedeutet,

oder dessen für Pflanzen verträgliches Säureadditionssalz oder Metallkomplex, außer den Verbindungen, in denen A unsubstituiertes Phenyl, B Alkyl, D Alkoxy, X N und n 0 bedeuten.

6. Verfahren zur Regulierung des Pflanzenwachstums, dadurch gekennzeichnet, daß man eine regulierend wirksame Menge eines Azolylethanderivates der Formel I

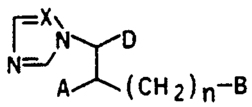

I

in welcher
A und B $C_1$-$C_8$-Alkyl, Phenyl, Biphenyl, Naphthyl, Benzyl, Tetrahydropyranyl, $C_3$-$C_8$-Cycloalkyl oder $C_5$-$C_8$-Cycloalkenyl bedeuten,
wobei diese Reste einfach bis dreifach durch Halogen, Nitro, Phenoxy, Amino, Alkyl, Alkoxy oder Halogenalkyl mit jeweils 1-bis 4-C-Atomen substituiert sein können,
D $C_1$-$C_4$-Alkoxy, Amino, gegebenenfalls durch $C_1$-$C_3$-Alkyl, $C_1$-$C_3$-Acyl substituiertes Amino, gegebenenfalls durch $C_1$-$C_4$-Alkyl substituiertes Mercapto, Thiophenyl, Aminophenyl, Hetaryloxy oder Phenoxy bedeutet,
wobei diese Reste einfach bis dreifach durch Halogen, Nitro, Phenoxy, Amino, Alkyl oder Halogenalkyl mit jeweils 1 bis 4 C-Atomen substituiert sein können,
n eine ganze Zahl von 1 bis 5 oder 0 bedeutet,
X den Rest CH oder N bedeutet,
oder dessen für Pflanzen verträgliches Säureadditionssalz oder Metallkomplex, außer den Verbindungen, in denen A unsubstituiertes Phenyl, B Alkyl, D Alkoxy, X N und n 0 bedeuten, auf Kulturpflanzen, Samen oder den Erdboden einwirken läßt

7. Verbindung der Formel I gemäß Anspruch 1, in der
A 2,4-Dichlorphenyl,
B Methyl,
D Methoxy,
n 1 und
X N
bedeutet.

8. Verbindung der Formel I gemäß Anspruch 1, in der
A 2,4-Dichlorphenyl,
B Methyl,
D Methoxy,
n 2 und
X N
bedeutet.

9. Verbindung der Formel I gemäß Anspruch 1, in der A 2,4-Dichlorphenyl,
B Methyl,
D Methoxy,
n 3 und
X N
bedeutet.

10. Verbindung der Formel I gemäß Anspruch 1, in der
A 2,4-Dichlorphenyl,
B Methyl,
D Ethoxy,
n 3 und
X N
bedeutet.

Tabelle (Fortsetzung)

| Bsp | A | B | D | n | X | Schmp./IR ($cm^{-1}$) | Isomer* |
|---|---|---|---|---|---|---|---|
| 303 | $4-Cl-C_6H_4$ | $2,4-Cl-C_6H_3$ | $OC_2H_5$ | 0 | N | | |
| 304 | $4-Cl-C_6H_4$ | $2-CH_3-C_6H_4$ | $OCH_3$ | 0 | N | | |
| 305 | $4-Cl-C_6H_4$ | $2-CH_3-C_6H_4$ | $OC_2H_5$ | 0 | N | | |
| 306 | $4-Cl-C_6H_4$ | $2,4-(CH_3)_2-C_6H_3$ | $OCH_3$ | 0 | N | | |
| 307 | $4-Cl-C_6H_4$ | $2,4-(CH_3)_2-C_6H_3$ | $OC_2H_5$ | 0 | N | | |
| 308 | $4-Cl-C_6H_4$ | $4-CF_3-C_6H_4$ | $OCH_3$ | 0 | N | | |
| 309 | $4-Cl-C_6H_4$ | $4-CF_3-C_6H_4$ | $OC_2H_5$ | 0 | N | | |
| 310 | $4-Cl-C_6H_4$ | $4-Br-C_6H_4$ | $OCH_3$ | 0 | N | | |
| 311 | $4-Cl-C_6H_4$ | $4-Br-C_6H_4$ | $OC_2H_5$ | 0 | N | | |
| 312 | $4-Cl-C_6H_4$ | $4-CH_3-C_6H_4$ | $OCH_3$ | 0 | N | | |
| 313 | $4-Cl-C_6H_4$ | $4-CH_3-C_6H_4$ | $OCH_3$ | 0 | CH | | |
| 314 | $4-Cl-C_6H_4$ | $4-CH_3-C_6H_4$ | $OC_2H_5$ | 0 | N | | |
| 315 | $4-CH_3-C_6H_4$ | $2-F-C_6H_4$ | $OCH_3$ | 0 | N | | |
| 316 | $4-CH_3-C_6H_4$ | $2-Cl-C_6H_4$ | $OCH_3$ | 0 | N | | |
| 317 | $4-CH_3-C_6H_4$ | $2,4-Cl_2-C_6H_3$ | $OCH_3$ | 0 | N | | |
| 318 | $4-CH_3-C_6H_4$ | $4-Br-C_6H_4$ | $OCH_3$ | 0 | N | | |
| 319 | $4-CH_3-C_6H_4$ | $4-CF_3-C_6H_4$ | $OCH_3$ | 0 | N | | |
| 320 | $4-CH_3-C_6H_4$ | $2-CH_3-C_6H_4$ | $OCH_3$ | 0 | N | | |
| 321 | $4-CH_3-C_6H_4$ | $4-CH_3-C_6H_4$ | $OCH_3$ | 0 | N | | |
| 322 | $4-CH_3-C_6H_4$ | $4-CH_3-C_6H_4$ | $OCH_3$ | 0 | CH | | |
| 323 | $4-CH_3-C_6H_4$ | $4-CH_3-C_6H_4$ | $OC_2H_5$ | 0 | N | | |
| 324 | $4-CH_3-C_6H_4$ | $4-CH_3-C_6H_4$ | $OC_2H_5$ | 0 | CH | | |
| 325 | $4-CH_3-C_6H_4$ | $4-CH_3-C_6H_4$ | $OCH(CH_3)_2$ | 0 | N | | |
| 326 | $4-CH_3-C_6H_4$ | $4-OCH_3-C_6H_4$ | $OCH_3$ | 0 | N | | |